# EUROPÄISCHE PATENTSCHRIFT

(11) **EP 4 076 607 B1**
(45) Veröffentlichungstag und Bekanntmachung des Hinweises auf die Patenterteilung: **03.05.2023**
(21) Anmeldenummer: 20824053.1
(22) Anmeldetag: 15.12.2020
(51) Int. Cl.: A61M 16/04, A61F 2/20

(54) **SPRECHVENTIL, VERSCHLUSSMITTEL FÜR EIN SPRECHVENTIL UND VERFAHREN**
SPEAKING VALVE, CLOSURE MEANS FOR A SPEAKING VALVE, AND METHOD
VALVE DE PHONATION, MOYEN DE FERMETURE POUR UNE VALVE DE PHONATION ET PROCÉDÉ

(30) Priorität: 16.12.2019 DE 102019134507
(43) Veröffentlichungstag der Anmeldung: 26.10.2022
(73) Patentinhaber: Andreas Fahl Medizintechnik-Vertrieb GmbH, 51149 Köln (DE)
(72) Erfinder: FAHL, Andreas, 51149 Köln (DE)
(74) Vertreter: Geskes, Christoph
(86) Internationale Anmeldenummer: PCT/EP2020/086202
(87) Internationale Veröffentlichungsnummer: WO 2021/122585

(56) Entgegenhaltungen:
- WO-A1-99/29268
- WO-A1-2016/115211
- US-A- 2 039 142
- US-A1- 2012 103 342
- US-A1- 2012 152 239
- US-A1- 2016 354 199

## Beschreibung

Die Erfindung betrifft ein Sprechventil zumindest umfassend ein Gehäuse und ein Verschlussmittel, ein Verschlussmittel für ein Sprechventil und ein Verfahren zur Einstellung eines Verschlussdruckes.

Sprechventile sind aus dem Stand der Technik hinlänglich bekannt. So beschreibt DE 10 2014 002 063 A1 ein Sprechventil mit einem Deckelteil, einem Stellmittel, einem Gehäuseteil und einem Filter, wobei das Gehäuseteil einen proximal in diesem angeordneten Ventilsitz zur Verfügung stellt, wobei das Stellmittel ein Verschlussteil umfasst und wobei das Verschlussteil bei Überführung des Sprechventiles aus einer Offenstellung in eine Geschlossenstellung mit dem Ventilsitz zusammenwirkt, wobei das Deckelteil zumindest teilweise aus einem elastischen Material gebildet ist, wobei durch eine Überführung des Sprechventiles aus der Offenstellung in die Geschlossenstellung eine zumindest teilweise Verformung zumindest eines Bereiches des Deckelteiles erfolgt, und wobei der Filter am Stellmittel derart angeordnet ist, dass der Filter der Bewegung des Stellmittels im Wesentlichen folgen kann.

WO 99/29268 A1 offenbart ein automatisches Sprechventil mit einem kalottenförmigen Ventil, dass an einem Ring aufgehängt ist.

Nachteilig an den bekannten Sprechventilen ist, dass diese manuell von einer Offenstellung in eine Geschlossenstellung überführt werden müssen, um einen Sprechvorgang einzuleiten. Somit ist kein natürlich anmutendes Sprechen möglich und der Benutzer ist insbesondere nicht in der Lage frei zu gestikulieren.

Aufgabe der Erfindung ist es daher, die aus dem Stand der Technik bekannten Sprechventile zu verbessern. Insbesondere ist die Aufgabe ein Sprechventil zur Verfügung zu stellen, mit dem ohne den Gebrauch der Hände ein Sprechvorgang eingeleitet werden kann.

Die Aufgabe wird erfindungsgemäß gelöst mittels eines Sprechventils zumindest umfassend ein Gehäuse und ein Verschlussmittel, wobei das Gehäuse ein Einsteckteil und ein haubenförmiges oder domförmiges Deckelteil mit einer Atemöffnung umfasst, wobei das Deckelteil auf dem Einsteckteil anordenbar ist, wobei das Verschlussmittel einen Außenring, ein Dichtmittel und zumindest eine Aufhängung umfasst, wobei das Dichtmittel mittels der Aufhängung am Außenring angeordnet ist, wobei eine Innenwandung des Deckelteils ein Deckelgewinde umfasst und das Deckelteil einen Ventilsitz zur Anlage des Dichtmittels ausbildet und mittels des Dichtmittels die Atemöffnung verschließbar ist.

Weiterhin wird die Aufgabe erfindungsgemäß gelöst mittels eines Verfahrens zur Einstellung eines Verschlussdruckes eines Sprechventils, umfassend ein Gehäuse und ein Verschlussmittel, wobei das Gehäuse ein Einsteckteil und ein haubenförmiges oder domförmiges Deckelteil mit einer Atemöffnung und einem Ventilsitz umfasst, wobei das Deckelteil auf das Einsteckteil anordenbar ist und eine Innenwandung des Deckelteils ein Deckelgewinde umfasst und wobei das Verschlussmittel einen Außenring, ein Dichtmittel und zumindest eine Aufhängung umfasst, wobei das Dichtmittel mittels der Aufhängung am Außenring angeordnet ist, wobei mittels Drehen des Deckelteils auf dem Einsteckteil ein Abstand zwischen Dichtmittel und dem Ventilsitz verändert wird.

Es wird ein Sprechventil zumindest umfassend ein Gehäuse und ein Verschlussmittel vorgeschlagen. Das Gehäuse umfasst ein Einsteckteil und ein haubenförmiges oder domförmiges Deckelteil mit einer Atemöffnung, wobei das Deckelteil auf dem Einsteckteil anordenbar ist, wobei das Verschlussmittel einen Außenring, ein Dichtmittel und zumindest eine Aufhängung umfasst, wobei das Dichtmittel mittels der Aufhängung am Außenring angeordnet ist, wobei eine Innenwandung des Deckelteils ein Deckelgewinde umfasst und das Deckelteil einen Ventilsitz zur Anlage des Dichtmittels ausbildet und mittels des Dichtmittels die Atemöffnung verschließbar ist.

Bei operativen Eingriffen im oberen Atemtrakt kann das Anlegen einer künstlichen Atemöffnung (Tracheostoma) in der Luftröhre notwendig sein, damit unter Umgehung von Mundraum und Kehlkopf Luft direkt in die Lunge eingeatmet werden kann. Beispielsweise bei laryngektomierten, also kehlkopflosen Personen, bei welchen der Kehlkopf operativ entfernt wurde, muss das Tracheostoma dauerhaft offen gehalten und stabilisiert werden, wozu insbesondere Trachealkanülen, in der Regel aus einer Außen- und einer Innenkanüle bestehend, in das Tracheostoma eingesetzt werden. Aber auch der Einsatz so genannter "Tracheostoma-Buttons" ist möglich, insbesondere für Personen, die keine Trachealkanüle mehr benötigen.

Schließlich können in das Tracheostoma auch Filtersysteme sowohl bei Tracheotomierten als auch bei Laryngektomierten eingesetzt werden. Derartige Filtersysteme bestehen insbesondere aus einem Pflaster mit einem eingesetzten Filter in einem Gehäuse oder aus einer üblicherweise selbstklebenden Basisplatte - in aller Regel aus Kunststoff gebildet, in welche Filter in einem Gehäuse unterschiedlichster Art einsetzbar sind. Zu den Filtersystemen, welche für laryngotracheale Hilfsmittel wie Trachealkanülen, Shunt-Ventile, Tracheostoma-Button und Filtersysteme mit Pflaster oder Basisplatte eingesetzt werden, zählen die gattungsgemäßen Wärme- und Feuchtigkeitsaustauscher, auch "künstliche Nase" genannt. Diese dienen dazu, die bei laryngektomierten und auch tracheotomierten Menschen fehlenden Regulationsmechanismen zur Erwärmung und Befeuchtung der Atemluft nachzubilden und ein In-Kontakt-Bringen der Luftröhre mit verstärkt trockener, kalter und nicht gefilterter Luft zu vermeiden. Denn durch die hierdurch hervorgerufene Reizung tritt eine erhöhte Schleimproduktion mit der nachfolgenden Gefahr der Verborkung auf. Durch Wärme- und Feuchtigkeitsaustauscher wird die eingeatmete Luft angefeuchtet, erwärmt und gleichzeitig gefiltert. Hierdurch wird die vorstehend erwähnte Borkenbildung weitgehend vermieden. Dabei hilft das regelmäßige Tragen der künstlichen Nase insbesondere bei starker Sekretabsonderung, da durch das Anfeuchten der Schleimhäute in der Luftröhre die Sekretproduktion vermindert wird.

Wärme- und Feuchtigkeitsaustauscher für Laryngektomierte und auch Tracheotomierte weisen ein Filtermaterial auf- in aller Regel aus Papier oder Schaumstoff gebildet - durch welches die ein- und ausgeatmete Luft geleitet wird. Beim Ausatmen hält das Filtermaterial Feuchtigkeit zurück, welche dann beim Einatmen in die Luftröhre transportiert wird. Aus dem Stand der Technik bekannte Wärme- und Feuchtigkeitsaustauscher gibt es in einer großen Vielzahl unterschiedlicher Ausbildungen für unterschiedliche Adapter. Ein Universaladapter gemäß DIN EN ISO 5356-1 umfasst einen Durchmesser von etwa 15 mm, es sind jedoch aus dem Stand der Technik auch weitere Adapter mit etwa 22 mm Durchmesser bekannt.

Ein weiteres Hilfsmittel für Laryngektomierte sind Stimmprothesen, die der Wiederherstellung der Stimme nach einer Laryngektomie dienen. Die Wiederherstellung der Stimme kann durch chirurgische Maßnahmen erfolgen. Wichtigstes Hilfsmittel dabei ist eine Stimmprothese, die auch als "Shunt-Ventil" bezeichnet wird. Mittels der Stimmprothese können Funktionen des entfernten Kehlkopfes ersetzt werden. Zum einen wird durch den Einsatz der Stimmprothese eine Luftzufuhr von der Lunge über die Speiseröhre (Ösophagus) in den Rachen ermöglicht. Die Ausatemluft wird dabei zum Sprechen verwendet. Zum anderen dichtet die Stimmprothese die Verbindung von der Speiseröhre zur Luftröhre (Trachea) beim Schlucken von Speisen und Getränken ab, wodurch der Nutzer der Stimmprothese vor einem versehentlichen Verschlucken der Nahrung geschützt ist.

Sind die Wunden nach dem operativen Eingriff geheilt, können die Menschen im Normalzustand nicht sprechen. Um ein Sprechen zu ermöglichen, ist bei Verwendung einer Stimmprothese oder der noch vorhandenen Larynx bei tracheotomierten Menschen der Luftaustritt am Hals zu verschließen, damit die Luft durch die Stimmprothese beziehungsweise die Larynx geleitet wird. Hierzu werden Sprechventile verwendet.

Das vorgeschlagene Sprechventil hat den Vorteil, dass dieses sich automatisch bei dem Vorhaben des Sprechens schließt. Dies wird insbesondere dadurch ermöglicht, dass natürlicherweise ein Ausatemdruck zur Einleitung des Sprechvorganges erhöht wird. Der erhöhte Druck deplatziert das Dichtmittel derart im Sprechventil, dass dieses zur Anlage an dem Ventilsitz kommen kann. Hierdurch wird die Atemöffnung verschlossen und die ausgeatmete Luft kann durch das Shunt-Ventil oder die gegebenenfalls noch vorhandene Larynx geleitet werden.

Das Sprechventil umfasst das Gehäuse. Das Gehäuse hat eine erste Seite und eine gegenüberliegende zweite Seite. Die erste Seite des Gehäuses ist bevorzugt distal anordenbar, so dass diese bei einer Benutzung von einer Körperoberfläche eines Benutzers weg weist. Die zweite Seite des Gehäuses ist bevorzugt proximal anordenbar, so dass diese bei der Benutzung zu der I<örperoberfläche des Benutzers weist.

Das Gehäuse umfasst des Weiteren das Einsteckteil. In einer Ausgestaltung umfasst das Einsteckteil eine erste Seite und einer der ersten Seite gegenüberliegende zweite Seite. Die erste Seite des Einsteckteils ist bevorzugt distal anordenbar, so dass diese bei einer Benutzung von einer Körperoberfläche eines Benutzers weg weist. Die zweite Seite des Einsteckteils ist bevorzugt proximal anordenbar, so dass diese bei der Benutzung zu der Körperoberfläche des Benutzers weist.

In einer Ausgestaltung umfasst das Einsteckteil einen Steckabschnitt. Der Steckabschnitt ist bevorzugt der zweiten Seite zugeordnet. Weiter umfasst der Steckabschnitt einen Konnektor für die Anbindung an ein laryngotracheales Hilfsmittel. In einer weiteren Ausgestaltung ist der Steckabschnitt als Konnektor ausgestaltet. Weiter bevorzugt ist vorgesehen, dass der Steckabschnitt einen Außendurchmesser von etwa 10 mm bis etwa 25 mm, bevorzugt etwa 15 mm oder etwa 22 mm umfasst.

Wird im Rahmen der Erfindung der Begriff "etwa" im Zusammenhang mit Werten oder Wertebereichen verwendet, so ist darunter ein Toleranzbereich zu verstehen, den der Fachmann auf diesem Gebiet für üblich erachtet, insbesondere ist ein Toleranzbereich von ±20 %, bevorzugt ±10 %, weiter bevorzugt ±5 % vorgesehen.

Soweit verschiedene Wertebereiche, beispielsweise bevorzugte und weiter bevorzugte Wertebereiche, in der vorliegenden Erfindung angegeben sind, sind die Untergrenzen und die Obergrenzen der verschiedenen Wertebereiche miteinander kombinierbar.

Ein laryngotracheales Hilfsmittel kann beispielsweise eine Trachealkanüle und/oder ein Tracheostomapflaster sein.

Beispielhafte Aufzählungen sind im Sinne der Erfindung als nicht abschließend anzusehen, sondern können im Rahmen des allgemeinen Fachwissens ergänzt werden.

In einer weiteren Ausgestaltung ist vorgesehen, dass das Einsteckteil einen Gewindeabschnitt umfasst. Der Gewindeabschnitt ist bevorzugt einer ersten Seite des Einsteckteils zugeordnet. Bevorzugt umfasst der Gewindeabschnitt eine Höhe, die sich in einer Längsrichtung des Einsteckteils erstreckt. Weiter bevorzugt umfasst der Gewindeabschnitt zumindest einen, bevorzugt genau einen Gewindegang. Weiter bevorzugt ist der Gewindeabschnitt als Außengewinde ausgestaltet. In einer Ausgestaltung umfasst der Gewindeabschnitt eine metrische Gewindesteigung von etwa 0,5 bis etwa 2-mal, bevorzugt etwa 0,7 bis etwa ein mal, weiter bevorzugt etwa 0,75-mal eine Höhe des Gewindeabschnittes.

Im Sinne der Erfindung ist eine metrische Gewindesteigung der Weg, den das Deckelteil der durch eine vollständige Umdrehung des Deckelteils auf dem Gehäuse in Längsrichtung zurücklegt. Insbesondere ist die metrische Gewindesteigung der Abstand zwischen zwei unmittelbar in Längsrichtung aufeinanderfolgenden Gewindespitzen eines Gewindes.

In einer weiteren Ausgestaltung umfasst das Einsteckteil eine Filterausnehmung. Die Filterausnehmung ist insbesondere ein zumindest teilweise von einer Innenwandung des Einsteckteils umschlossener Raum. Insbesondere ist die Filterausnehmung eine Ausnehmung zur Aufnahme eines insbesondere unten beschriebenen Filters insbesondere in dem Einsteckteil. Weiter bevorzugt ist das Einsteckteil im Wesentlichen als Zylinder ausgestaltet. Weiter bevorzugt umfasst das Einsteckteil eine zylindrische Ausnehmung, die als Filterausnehmung angesprochen werden kann. Insbesondere wird die Filterausnehmung durch die im Wesentlichen zylindrische Innenwandung zumindest radial begrenzt. Die zylindrische Innenwandung ist insbesondere eine innenseitige Oberfläche des Einsteckteils. In einer weiteren Ausgestaltung umfasst die zylindrische Innenwandung eine Filterarretierung. In einer Ausgestaltung ist vorgesehen, dass das Sprechventil den Filter umfasst, der in dem Einsteckteil angeordnet ist, wobei das Einsteckteil die Filterarretierung umfasst, mit der der Filter in dem Einsteckteil gehalten ist. Die Filterarretierung ist insbesondere derart ausgestaltet, dass ein in dem Einsteckteil angeordneter Filter im Wesentlichen nicht in Richtung der ersten Seite bewegt wird, weiter bevorzugt nicht mit dem Verschlussmittel in Kontakt kommt, weiter bevorzugt das Verschlussmittel nicht in seiner Funktion behindert, wenn ein Druck, bevorzugt ein Atemdruck auf den Filter wirkt. Die Filterarretierung kann beispielsweise als nach radial innen ausgeprägte Materialanhäufung ausgestaltet sein. In einer weiteren Ausgestaltung umfasst die Filterarretierung zumindest einen Haken. In einer weiteren Ausgestaltung ist die Filterausnehmung zur zweiten Seite hin durch einen Respirationsschutz begrenzt. Der Respirationsschutz verhindert bevorzugt ein Einatmen eines in dem Einsteckteil befindlichen Filters durch den Benutzer. Insbesondere ist in einer Ausgestaltung vorgesehen, dass der Respirationsschutz zumindest eine Strebe oder ein Gitter umfasst.

Der Begriff "im Wesentlichen" gibt einen Toleranzbereich an, der für den Fachmann unter wirtschaftlichen und technischen Gesichtspunkten zu vertreten ist, so dass das entsprechende Merkmal noch als solches zu erkennen oder verwirklicht ist.

In einer weiteren Ausgestaltung ist vorgesehen, dass das Einsteckteil einen Anschlag umfasst. Insbesondere kann der Anschlag dazu verwendet werden, ein Einstecken des Steckabschnittes in ein laryngotracheale Hilfsmittel zu begrenzen. Der Anschlag kann beispielsweise als zumindest teilweise umlaufender Kragen ausgestaltet sein. In einer weiteren Ausgestaltung ist der Anschlag als zumindest eine Materialanhäufung auf dem Außenumfang des Einsteckteils ausgestaltet. Insbesondere ist der Anschlag der ersten Seite des Steckabschnittes angeordnet. Weiter bevorzugt ist der Anschlag zwischen dem Gewindeabschnitt und dem Einsteckteil angeordnet.

In einer alternativen Ausgestaltung ist vorgesehen, dass das Deckelteil mittels eines Bajonettverschlusses auf dem Einsteckteil anordenbar ist.

In einer weiteren Ausgestaltung umfasst das Gehäuse, weiter bevorzugt das Einsteckteil oder das Deckelteil eine Verschlussmittelaufnahme. In einer Ausgestaltung ist die Verschlussmittelaufnahme insbesondere der ersten Seite des Einsteckteils zugeordnet. In einer Ausgestaltung ist die Verschlussmittelaufnahme insbesondere der zweiten Seite des Deckelteils zugeordnet. Bevorzugt ist die Verschlussmittelaufnahme an der zylindrischen Innenwandung des Einsteckteils oder der Innenwandung des Deckelteils angeordnet oder wird durch diese gebildet. In einer Ausgestaltung ist vorgesehen, dass die Verschlussmittelaufnahme als eine zumindest teilweise umlaufende, bevorzugt vollständig umlaufende Kerbe, bevorzugt in der zylindrischen Innenwandung des Einsteckteils oder der Innenwandung des Deckelteils, ausgestaltet ist. In einer weiteren Ausgestaltung ist vorgesehen, dass die Verschlussmittelaufnahme Rastmittel umfasst, bevorzugt als Materialanhäufung ausgebildete Rastmittel umfasst. In einer weiteren Ausgestaltung ist vorgesehen, dass die Verschlussmittelaufnahme zumindest einen zumindest teilweise umlaufenden Wulst insbesondere auf der zylindrischen Innenwandung des Einsteckteils oder der Innenwandung des Deckelteils umfasst. Weiter bevorzugt sind zwei in Längsrichtung des Gehäuses voneinander beabstandete, zumindest teilweise umlaufende Wulste vorgesehen, die als Verschlussmittelaufnahme angesprochen werden können. Bevorzugt ist die Verschlussmittelaufnahme dazu ausgestaltet das Verschlussmittel in oder an dem Gehäuse im Wesentlichen zu fixieren, bevorzugt von dem Filter beabstandet zu halten, weiter bevorzugt, insbesondere bei einem Ausatemdruck, eine Bewegung des Verschlussmittels in Richtung der ersten Seite zu begrenzen.

Das Gehäuse umfasst des Weiteren den Deckel. Erfindungsgemäß ist das Deckelteil haubenförmig oder domförmig ausgestaltet. In einer weiteren Ausgestaltung ist das Deckelteil in Form einer Überwurfmutter ausgestaltet. Insbesondere umfasst das Deckelteil eine erste Seite und eine der ersten Seite gegenüberliegende zweite Seite. Bevorzugt ist die erste Seite bei einer Benutzung des Sprechventils distal anordenbar und die zweite Seite proximal anordenbar.

Das Deckelteil umfasst insbesondere einen Mantel. Das Deckelteil umfasst eine Innenwandung, die bevorzugt eine Innenoberfläche des Mantels ist. Weiterhin bevorzugt umfasst das Deckelteil eine Ringwandung. Die Ringwandung ist bevorzugt der ersten Seite des Deckelteils zugeordnet. Weiterhin umfasst eine der ersten Seite zugewandte Oberfläche der Ringwandung einen Flächenvektor, der im Wesentlichen in Richtung der ersten Seite gerichtet ist. Insbesondere wird durch die Ringwandung eine Atemöffnung gebildet. Bevorzugt ist vorgesehen, dass die Ringwandung an den Mantel grenzt oder in diesen übergeht. Insbesondere ist ein Übergangsbereich zwischen Mantel und Ringwandung als Fase und/oder Radius ausgebildet. In einer weiteren Ausgestaltung ist vorgesehen, dass die Ringwandung, insbesondere auf der zweiten Seite, einen Ventilsitz bildet. Der Ventilsitz ist insbesondere eine Oberfläche auf der zweiten Seite der Ringwandung, die einen Flächenvektor umfasst, der in Richtung der zweiten Seite gerichtet ist. Der Ventilsitz ist insbesondere derart ausgebildet, dass dieser mit dem Verschlussteil zusammen wirken kann.

Das Deckelteil umfasst das Deckelgewinde. Insbesondere ist vorgesehen, dass das Deckelgewinde als Innengewinde ausgestaltet ist. Erfindungsgemäß ist vorgesehen, dass das Deckelgewinde, das bevorzugt als Innengewinde ausgestaltet ist, an der Innenwandung des Deckelteils angeordnet ist. In einer Ausgestaltung umfasst die Innenwandung des Deckelteils das Deckelgewinde. In einer weiteren Ausgestaltung ist vorgesehen, dass das Deckelgewinde eine metrische Gewindesteigung von etwa 0,5 bis etwa 2 Mal, bevorzugt etwa 0,7 bis etwa 1 Mal, weiter bevorzugt etwa 0,75 Mal einer Höhe des Deckelgewindes umfasst.

Bevorzugt sind der Gewindegang des Einsteckteils und das Deckelgewinde aufeinander abgestimmt. In einer Ausgestaltung ist vorgesehen, dass der Gewindegang und das Deckelgewinde etwa 0,5 Umdrehungen bis etwa 2 Umdrehungen, bevorzugt etwa 0,7 Umdrehungen bis etwa eine Umdrehung, weiter bevorzugt etwa 0,75 Umdrehungen aufweisen. In einer Ausgestaltung ist vorgesehen, dass der Gewindegang und das Deckelgewinde eine metrische Gewindesteigung von etwa 0,5 bis etwa 2, bevorzugt etwa 0,7 bis etwa 1, weiter bevorzugt etwa 0,75 Mal einer Höhe des Gewindeabschnittes und/oder des Deckelgewindes ist.

In einer Ausgestaltung ist vorgesehen, dass das Einsteckteil einen Gewindeabschnitt mit einem Gewindegang umfasst und das Deckelteil ein Deckelgewinde umfasst, wobei der Gewindegang des Einsteckteils und das Deckelgewinde miteinander korrespondierend ausgestaltet sind. In einer weiteren Ausgestaltung ist vorgesehen, dass das Deckelgewinde und/oder der Gewindeabschnitt des Einsteckteils einen Anschlag aufweisen, der bevorzugt eine Drehung des Deckelteils auf dem Einsteckteil begrenzt. Der Anschlag kann beispielsweise einem Ende des Deckelgewindes und/oder einem Ende des Gewindeabschnitts zugeordnet sein. In einer Ausgestaltung ist vorgesehen, dass der Anschlag mittels einer definierten Kraft beziehungsweise eines definierten Moments überwindbar ist. Beispielsweise kann das Deckelteil auf das Einsteckteil durch Anwendung eines erhöhten Momentes auf das Einsteckteil geschraubt werden, bevorzugt bis der Anschlag überwunden und weiter bevorzugt das Deckelteil auf dem Einsteckteil eingerastet ist. In einer Ausgestaltung ist beispielsweise vorgesehen, dass das Deckelteil mit einem geringeren Moment aufschraubbar als abschraubbar ist. Insbesondere umfasst der Anschlag des Deckelteils und/oder des Einsteckteils zumindest eine schiefe Ebene. Insbesondere ist eine Geometrie des Anschlages des Deckelteils und/oder des Einsteckteils vorgesehen, der bei beim Aufschrauben eine flachere schiefe Ebene überwunden werden muss, als beim Abschrauben. In einer bevorzugten Ausgestaltung rastet das Deckelteil nach Überwindung des Anschlages mit einem hörbaren Geräusch auf dem Einsteckteil auf. Der Anschlag ist bevorzugt vorgesehen, um eine versehentliche Demontage beim Drehen des Deckelteils auf dem Einsteckteil zu gewährleisten.

In einer Ausgestaltung ist vorgesehen, dass das Sprechventil den Filter umfasst. Der Filter ist bevorzugt in einer Ausführungsform vorgesehen, in der das Sprechventil auch als Wärme-Feuchtigkeits-Austauscher ausgestaltet ist. Der Filter kann bevorzugt Wärme und Feuchtigkeit aus der ausgeatmeten Luft speichern und beim Einatmen an die eingeatmete Luft abgeben. In einer Ausgestaltung umfasst der Filter eine erste Seite und eine der ersten Seite gegenüberliegende zweite Seite. Bevorzugt ist die erste Seite bei einer Benutzung des Sprechventils distal anordenbar und die zweite Seite proximal anordenbar. In einer Ausgestaltung ist der Filter zylindrisch ausgestaltet. Insbesondere ist der Filter in der Filterausnehmung des Einsteckteils anordenbar. In einer Ausgestaltung umfasst der Filter zumindest ein Material ausgesucht aus einer Gruppe umfassend Papier, Polyurethan, Polyethylen, Polypropylen und/oder Biopolymere.

Das Sprechventil umfasst das Verschlussmittel. Das Verschlussmittel umfasst eine erste Seite und eine der ersten Seite gegenüberliegende zweite Seite. Bevorzugt ist die erste Seite bei einer Benutzung des Sprechventils distal anordenbar und die zweite Seite proximal anordenbar.

In einer Ausgestaltung ist vorgesehen, dass das Verschlussmittel zumindest teilweise in einer zumindest teilweise umlaufenden Verschlussmittelaufnahme des Gehäuses anordenbar ist. Insbesondere ist zwischen der Verschlussmittelaufnahme und dem Verschlussmittel eine formschlüssige Verbindung herstellbar. Insbesondere wird das Verschlussmittel in die Verschlussmittelaufnahme eingerastet.

Das Verschlussmittel ist insbesondere dafür vorgesehen, die Atemöffnung des Deckelteils zu verschließen. Weiter bevorzugt erlaubt es das Verschlussmittel, dass bei einem definierten Ausatemdruck die Atemöffnung mittels des Verschlussmittels verschlossen wird, insbesondere ohne einen manuellen Eingriff des Benutzers.

Das Verschlussmittel umfasst den Außenring. Bevorzugt ist der Außenring vollständig geschlossen. Der Außenring umfasst insbesondere einen Ringmanteloberfläche. Die Ringmanteloberfläche ist insbesondere eine radiale Oberfläche des Außenrings. In einer Ausgestaltung umfasst die Ringmanteloberfläche zumindest eine zumindest teilweise umlaufende Materialanhäufung. Insbesondere ist die Materialanhäufung in Form eines Wulstes ausgestaltet. In einer weiteren Ausgestaltung ist vorgesehen, dass die Materialanhäufung derart ausgestaltet, dass diese mit der Verschlussmittelaufnahme zusammen wirken kann, insbesondere in diese einrastbar ist.

Das Verschlussmittel umfasst das Dichtmittel. Das Dichtmittel ist insbesondere zentral, bevorzugt konzentrisch im Außenring angeordnet. Weiter bevorzugt ist das Dichtmittel im Wesentlichen kreisförmig ausgestaltet. In einer Ausgestaltung ist vorgesehen, dass das Dichtmittel vollflächig geschlossen ist, insbesondere ist das Dichtmittel als Kreisscheibe ausgestaltet. Bevorzugt umfasst das Dichtmittel einen größeren Radius auf als die Atemöffnung des Deckelteils. In einer weiteren Ausgestaltung ist das Dichtmittel derart ausgestaltet, dass dieses zumindest teilweise auf dem Ventilsitz des Deckelteils anlegbar ist. Bevorzugt wirkt das Dichtmittel mit dem Ventilsitz zusammen, um die Atemöffnung zu verschließen.

Bevorzugt ist das Dichtmittel mittels zumindest einer Aufhängung an dem Außenring angeordnet. Insbesondere ist die zumindest eine Aufhängung zwischen Außenring und Dichtmittel angeordnet. Weiter bevorzugt umfasst das Verschlussmittel etwa eine bis etwa 12, bevorzugt etwa 3 bis etwa 8, weiter bevorzugt etwa 3 bis etwa 4, weiter bevorzugt genau 4 Aufhängungen. In einer Ausgestaltung ist vorgesehen, dass die zumindest eine Aufhängung stegförmig ausgestaltet ist. Insbesondere kann die zumindest eine Aufhängung geschlitzt oder ungeschlitzt ausgeführt sein. Ist die Aufhängung geschlitzt ausgeführt umfasst diese insbesondere zentral eine oder mehrere insbesondere von der ersten Seite zur zweiten Seite vollständig durchgängige Ausnehmungen, die sich weiter bevorzugt in Längsrichtung der Aufhängung erstrecken. In einer weiteren Ausgestaltung ist vorgesehen, dass die zumindest eine Aufhängung mit dem Außenring materialverbunden ist. In einer weiteren Ausgestaltung ist vorgesehen, dass die zumindest eine Aufhängung mit dem Dichtmittel materialverbunden ist. In einer weiteren Ausgestaltung ist vorgesehen, dass die Aufhängung an dem Dichtmittel und/oder an dem Außenring angeklebt, angespritzt oder in sonst einer Weise befestigt ist.

In einer Ausgestaltung ist vorgesehen, dass mittels der Aufhängung das Dichtmittel gummielastisch am Außenring aufgehängt ist. Bevorzugt umfasst das Verschlussmittel, weiter bevorzugt der Außenring, das Dichtmittel und/oder die zumindest eine Aufhängung zumindest ein Material ausgewählt aus einer Gruppe zumindest umfassend thermoplastische Elastomere, Silikon und/oder Kautschuk.

Vorteilhafter Weise ist das Verschlussmittel derart im Gehäuse angeordnet, dass ein definierter Ausatemdruck das Dichtmittel in Richtung der ersten Seite, insbesondere in Richtung des Ventilsitzes drückt. In der Regel ist der Ausatemdruck bei Einleitung eines Sprechvorganges größer als beim normalen Atmen. Insbesondere wird das Dichtmittel bei Einleitung eines Sprechvorganges beziehungsweise einem erhöhten Atemdruck derart deplatziert, dass die Atemöffnung durch dieses verschlossen wird. Die Ausatemluft kann somit beispielsweise durch eine Stimmprothese in den Ösophagus oder durch den Larynx geleitet werden und somit ist dem Benutzer ermöglicht, ohne manuelle Betätigung des Sprechventiles zu sprechen. Insbesondere ist das Sprechventil derart einstellbar, dass beim Ausatemdruck für die Einleitung eines Sprechvorganges das Dichtmittel dichtend auf dem Ventilsitz anliegt und die Atemöffnung verschlossen wird. Da der Ausatemdruck zum Sprechen bei jeder Person individuell ist und je nach Betätigung des Benutzers der Ausatemdruck beim normalen Atem auch variieren kann, kann das vorgeschlagene Sprechventil vorteilhaft mittels Drehen des Deckelteils auf dem Einsteckteil eingestellt werden.

In einer Ausgestaltung ist vorgesehen, dass mittels Drehen des Deckelteils auf dem Einsteckteil ein Abstand des Verschlussmittels von dem Ventilsitz veränderbar ist. Durch die Drehung des Deckelteils auf dem Einsteckteil wird das Deckelteil weiter auf oder abgeschraubt, so dass ein Abstand zwischen Ventilsitz und Dichtmittel in einer neutralen Position verändert werden kann. Die neutrale Position des Dichtmittels ist insbesondere dann gegeben, wenn in dem Sprechventil kein Ausatemdruck oder Einatemdruck vorliegt und das Dichtmittel zentral im Außenring gehalten ist. Wird beispielsweise das Deckelteil vollständig aufgeschraubt, ist der Abstand zwischen Ventilsitz und Dichtmittel gering. Dann ist nur ein geringer Ausatemdruck notwendig, um das Dichtmittel ausreichend weit aus der neutralen Position zu deplatzieren, damit dieses an dem Ventilsitz anliegt. Wird beispielsweise das Deckelteil fast vollständig abgeschraubt, insbesondere bis zu einem Anschlag abgeschraubt, ist ein erhöhter Ausatemdruck notwendig, um das Dichtmittel ausreichend weit aus der neutralen Position zu deplatzieren, damit dieses an dem Ventilsitz anliegt. Gegebenenfalls ist das Deckelteil so weit abschraubbar, insbesondere der Abstand zwischen Ventilsitz und Dichtmittel in der neutralen Position derart einstellbar, dass kein von dem Benutzer ausgeübter Ausatemdruck das Dichtmittel zur Anlage an den Ventilsitz bringen kann. Dies kann insbesondere bei sportlichen Aktivitäten gewünscht sein. Soll dann wieder das Sprechen ermöglicht werden, so kann der Benutzer das Deckelteil weiter auf das Einsteckteil aufdrehen, bis der Abstand zwischen Ventilsitz und Dichtmittel in der neutralen Position ausreichend nah ist, um einen Sprechvorgang einzuleiten.

In einer beispielhaften Ausgestaltung des Sprechventils umfasst dieses ein Gehäuse mit einem Deckelteil und einem Einsteckteil. Das Deckelteil umfasst eine erste Seite und eine dieser gegenüber liegende zweite Seite. Die erste Seite ist im Gebrauch des Sprechventils distal anordenbar. Die zweite Seite ist im Gebrauch des Sprechventils proximal anordenbar. Das Deckelteil umfasst einen Mantel, der auf seiner Innenseite ein Deckelgewinde umfasst. Das Deckelgewinde ist beispielhaft ein eingängiges Innengewinde mit einer Steigung von etwa 0,75 der Höhe des Deckelgewindes. Weiterhin umfasst beispielhaft das Deckelteil eine Ringwandung, die über einen als Fase ausgestalteten Übergang mit dem Mantel verbunden ist. Die Ringwandung umfasst zentral eine Atemöffnung. Weiterhin umfasst die Ringwandung einen Ventilsitz, der auf der zweiten Seite der Ringwandung angeordnet ist. Der Ventilsitz umgibt die Atemöffnung.

Das beispielhafte Gehäuse umfasst des Weiteren eine das Einsteckteil. Diese umfasst eine erste Seite und eine dieser gegenüber liegende zweite Seite. Die erste Seite ist im Gebrauch des Sprechventils distal anordenbar. Die zweite Seite ist im Gebrauch des Sprechventils proximal anordenbar. Das Einsteckteil umfasst einen Steckabschnitt und einen Gewindeabschnitt. Der Gewindeabschnitt umfasst einen Gewindegang mit einer Höhe und einer metrischen Gewindesteigung von beispielhaft etwa 0,75-mal der Höhe. Der zweiten Seite zugeordnet umfasst das Einsteckteil einen Steckabschnitt, der die Funktion eines 15 mm oder 22 mm Konnektors übernimmt. Mit dem Steckabschnitt ist das Sprechventil beispielhaft mit einem nicht dargestellten Tracheostomahilfsmittel verbindbar. Zwischen dem Steckabschnitt und dem Gewindeabschnitt ist ein Anschlag angeordnet, der sowohl ein Aufschrauben des Deckelteils auf den Gewindeabschnitt als auch ein Einstecken des Steckabschnittes in ein Tracheostomahilfsmittel begrenzt.

Die beispielhafte Einsteckteil umfasst eine Filterausnehmung, in die ein Filter aufgenommen werden kann. Die Filterausnehmung ist zur zweiten Seite hin durch einen Respirationsschutz begrenzt. Der Respirationsschutz umfasst beispielhaft eine Anzahl von Stegen, die ein Einatmen des Filters verhindern. Der ersten Seite zugeordnet ist eine Verschlussmittelaufnahme in einer die Filterausnehmung begrenzenden Innenwandung angeordnet, die als umlaufende Kerbe ausgestaltet ist. Ein Verschlussmittel kann in die Verschlussmittelaufnahme einrasten. Hierzu umfasst das Verschlussmittel an einem Außenring beispielhaft einen Wulst.

Wird in der beispielhaften Ausführungsform das Deckelteil auf das Einsteckteil geschraubt, so greift der Gewindeabschnitt in das Deckelgewinde ein. Mittels Drehen des Deckelteils auf dem Einsteckteil kann folglich ein Abstand des Ventilsitzes zum Verschlussmittel, das in dem Einsteckteil arretiert ist, verändert werden. Beispielhaft kann der Gewindeabschnitt und/oder das Deckelgewinde einen Anschlag aufweisen, der ein Drehen des Deckelteils auf dem Einsteckteil begrenzt und insbesondere ein versehentliches Trennen des Deckelteils von dem Einsteckteil verhindert.

Das beispielhafte Verschlussmittel umfasst eine erste Seite und eine zweite Seite. Die erste Seite ist im Gebrauch des Sprechventils distal anordenbar. Die zweite Seite ist im Gebrauch des Sprechventils proximal anordenbar. Das Verschlussmittel umfasst einen Außenring, der auf seinem Ringmantel einen umlaufenden Wulst umfasst. Ein Dichtmittel ist in einer neutralen Position konzentrisch im Außenring angeordnet. Gehalten wird das Dichtmittel von beispielhaft vier Aufhängungen. In weiteren beispielhaften Ausgestaltungen können auch mehr oder weniger Aufhängungen vorgesehen sein.

Im Gebrauch des beispielhaften Sprechventils wird beim Ausatmen ein Druck von der zweiten Seite aufgebaut, der das Dichtmittel zur ersten Seite hin deplatziert. Diese Deplatzierung ist größer, wenn der Benutzer versucht zu sprechen, da natürlicherweise der Ausatemdruck beim Sprechen größer ist. Ist das Deckelteil so weit auf das Einsteckteil aufgeschraubt, dass das Dichtmittel dabei zur Anlage an den Ventilsitz kommt, wird das Sprechventil verschlossen und die ausgeatmete Luft kann durch die Larynx des Benutzers oder ein Shunt-Ventil strömen, damit der Benutzer sprechen kann.

Weiterhin wird ein Verschlussmittel für ein oben beschriebenes Sprechventil vorgeschlagen. Das Verschlussmittel umfasst einen Außenring, ein Dichtmittel und zumindest eine Aufhängung, wobei das Dichtmittel mittels der Aufhängung am Außenring angeordnet ist. Insbesondere kann das Verschlussmittel wie oben beschrieben ausgestaltet sein.

Das Verschlussmittel umfasst eine erste Seite und eine der ersten Seite gegenüberliegende zweite Seite. Bevorzugt ist die erste Seite bei einer Benutzung des Sprechventils distal anordenbar und die zweite Seite proximal anordenbar.

Das Verschlussmittel ist insbesondere dafür vorgesehen, die Atemöffnung des oben beschriebenen Deckelteils zu verschließen. Weiter bevorzugt erlaubt es das Verschlussmittel, dass bei einem definierten Ausatemdruck die Atemöffnung mittels des Verschlussmittels verschlossen wird, insbesondere ohne einen manuellen Eingriff des Benutzers.

Das Verschlussmittel umfasst den Außenring. Bevorzugt ist der Außenring vollständig geschlossen. Der Außenring umfasst insbesondere einen Ringmanteloberfläche. Die Ringmanteloberfläche ist insbesondere eine radiale Oberfläche des Außenrings. In einer Ausgestaltung umfasst die Ringmanteloberfläche zumindest eine zumindest teilweise umlaufende Materialanhäufung. Insbesondere ist die Materialanhäufung in Form eines Wulstes ausgestaltet. In einer weiteren Ausgestaltung ist vorgesehen, dass die Materialanhäufung derart ausgestaltet, dass diese mit der Verschlussmittelaufnahme zusammen wirken kann, insbesondere in diese einrastbar ist.

Das Verschlussmittel umfasst das Dichtmittel. Das Dichtmittel ist insbesondere zentral, bevorzugt konzentrisch im Außenring angeordnet. Weiter bevorzugt ist das Dichtmittel im Wesentlichen kreisförmig ausgestaltet. In einer Ausgestaltung ist vorgesehen, dass das Dichtmittel vollflächig geschlossen ist, insbesondere ist das Dichtmittel als Kreisscheibe ausgestaltet. Bevorzugt umfasst das Dichtmittel einen größeren Radius auf als die Atemöffnung des Deckelteils. In einer weiteren Ausgestaltung ist das Dichtmittel derart ausgestaltet, dass dieses zumindest teilweise auf dem Ventilsitz des Deckelteils anlegbar ist. Bevorzugt wirkt das Dichtmittel mit dem Ventilsitz zusammen, um die Atemöffnung zu verschließen.

Bevorzugt ist das Dichtmittel mittels zumindest einer Aufhängung an dem Außenring angeordnet. Insbesondere ist die zumindest eine Aufhängung zwischen Außenring und Dichtmittel angeordnet. Weiter bevorzugt umfasst das Verschlussmittel etwa eine bis etwa 12, bevorzugt etwa 3 bis etwa 8, weiter bevorzugt etwa 3 bis etwa 4, weiter bevorzugt genau 4 Aufhängungen. In einer Ausgestaltung ist vorgesehen, dass die zumindest eine Aufhängung stegförmig ausgestaltet ist. Insbesondere kann die zumindest eine Aufhängung geschlitzt oder ungeschlitzt ausgeführt sein. Ist die Aufhängung geschlitzt ausgeführt umfasst diese insbesondere zentral eine oder mehrere insbesondere von der ersten Seite zur zweiten Seite vollständig durchgängige Ausnehmungen, die sich weiter bevorzugt in Längsrichtung der Aufhängung erstrecken. In einer weiteren Ausgestaltung ist vorgesehen, dass die zumindest eine Aufhängung mit dem Außenring materialverbunden ist. In einer weiteren Ausgestaltung ist vorgesehen, dass die zumindest eine Aufhängung mit dem Dichtmittel materialverbunden ist. In einer weiteren Ausgestaltung ist vorgesehen, dass die Aufhängung an dem Dichtmittel und/oder an dem Außenring angeklebt, angespritzt oder in sonst einer Weise befestigt ist.

In einer Ausgestaltung ist vorgesehen, dass mittels der Aufhängung das Dichtmittel gummielastisch am Außenring aufgehängt ist. Bevorzugt umfasst das Verschlussmittel, weiter bevorzugt der Außenring, das Dichtmittel und/oder die zumindest eine Aufhängung zumindest ein Material ausgewählt aus einer Gruppe zumindest umfassend thermoplastische Elastomere, Silikon und/oder Kautschuk.

Vorteilhafter Weise ist das Verschlussmittel derart im Gehäuse angeordnet, dass ein definierter Ausatemdruck das Dichtmittel in Richtung der ersten Seite, insbesondere in Richtung des Ventilsitzes drückt. In der Regel ist der Ausatemdruck bei Einleitung eines Sprechvorganges größer als beim normalen Atmen. Insbesondere ist das Dichtmittel bei Einleitung eines Sprechvorganges beziehungsweise einem erhöhten Atemdruck deplatzierbar.

Ein beispielhaftes Verschlussmittel umfasst eine erste Seite und eine zweite Seite. Die erste Seite ist im Gebrauch des Sprechventils distal anordenbar. Die zweite Seite ist im Gebrauch des Sprechventils proximal anordenbar. Das Verschlussmittel umfasst einen Außenring, der auf seinem Ringmantel einen umlaufenden Wulst umfasst. Ein Dichtmittel ist in einer neutralen Position konzentrisch im Außenring angeordnet. Gehalten wird das Dichtmittel von beispielhaft vier Aufhängungen. In weiteren beispielhaften Ausgestaltungen können auch mehr oder weniger Aufhängungen vorgesehen sein.

Weiterhin wird ein Verfahren zur Einstellung eines Verschlussdruckes eines oben beschriebenen Sprechventils vorgeschlagen. Das Sprechventil umfasst ein Gehäuse und ein Verschlussmittel, wobei das Gehäuse ein Einsteckteil und ein haubenförmiges oder domförmiges Deckelteil mit einer Atemöffnung und einem Ventilsitz umfasst. Das Deckelteil ist auf das Einsteckteil anordenbar und eine Innenwandung des Deckelteils umfasst ein Deckelgewinde. Das Verschlussmittel umfasst einen Außenring, ein Dichtmittel und zumindest eine Aufhängung, wobei das Dichtmittel mittels der Aufhängung am Außenring angeordnet ist. Mittels Drehen des Deckelteils auf dem Einsteckteil wird ein Abstand zwischen Dichtmittel und dem Ventilsitz verändert.

In einer beispielhaften Ausgestaltung des Verfahrens wird das Deckelteil zumindest so weit gedreht, dass ein Abstand zwischen dem Verschlussmittel, insbesondere dem Dichtmittel derart ist, dass ein Ausatemdruck das Dichtmittel nicht gegen den Ventilsitz zur Anlage bringt. Dies ist insbesondere dann von Vorteil, wenn der Benutzer nicht sprechen will und insbesondere verhindern will, dass Luft durch das Shunt-Ventil strömt, beispielsweise beim Sport.

In einer weiteren beispielhaften Ausgestaltung des Verfahrens wird das Deckelteil zumindest so weit gedreht, dass ein Abstand zwischen dem Verschlussmittel, insbesondere dem Dichtmittel derart ist, dass ein Ausatemdruck das Dichtmittel gegen den Ventilsitz zur Anlage bringt. Insbesondere kann der Benutzer das Deckelteil genau so weit auf das Einsteckteil aufschrauben, dass eine Deplatzierung des Dichtmittels beim Ausatmen bei einer Intention zu sprechen ausreicht, um zur Anlage an den Ventilsitz zu kommen. Vorzugsweise versucht der Benutzer zu sprechen und schraubt dabei das Deckelteil so lange auf das Einsteckteil auf, bis die Atemöffnung verschlossen ist. Sollte die Atemöffnung ungewollt verschlossen werden schraubt der Benutzer vorzugsweise das Deckelteil wieder so weit ab, bis dies beim der gegebenen Situation normalen atmen in nicht mehr geschieht.

Weitere vorteilhafte Ausgestaltungen gehen aus den nachfolgenden Zeichnungen hervor. Die dort dargestellten Weiterbildungen sind jedoch nicht beschränkend auszulegen, vielmehr können die dort beschriebenen Merkmale untereinander und mit den oben beschriebenen Merkmalen zu weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen. Gleiche Teile oder Teile mit gleicher Funktion weisen im Folgenden die gleichen Bezugszeichen auf. Es zeigen:
Fig. 1 eine Explosionsansicht eines Sprechventils; und
Fig. 2 ein Verschlussmittel in einer Detailansicht;

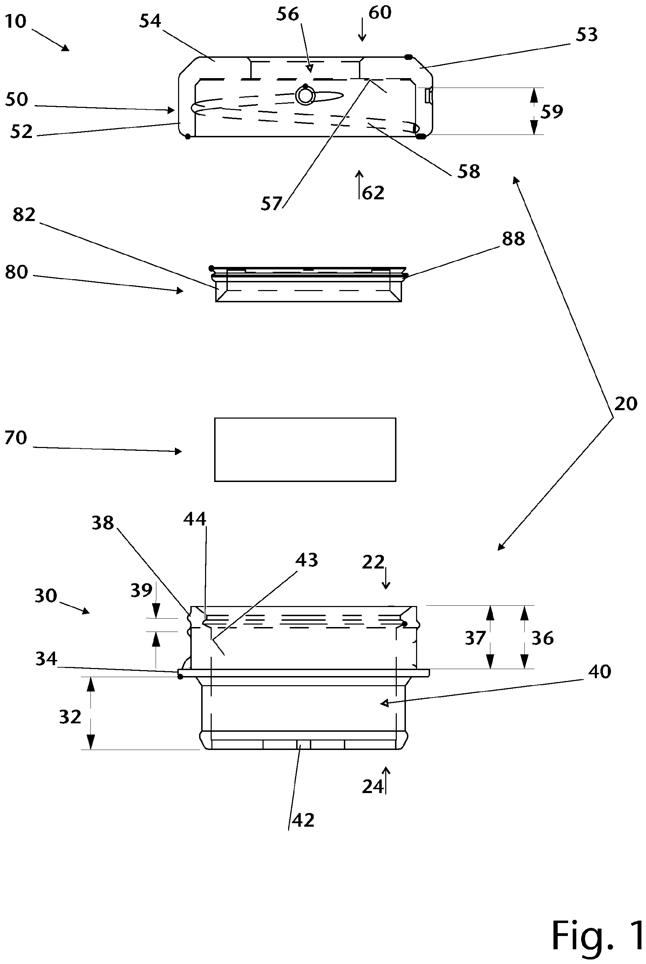

Fig. 1 zeigt eine Explosionsansicht eines Sprechventils 10. Dieses umfasst ein Gehäuse 20 mit einem Deckelteil 50 und einem Einsteckteil 30. Das Deckelteil 50 umfasst eine erste Seite 60 und eine dieser gegenüber liegende zweite Seite 62. Die erste Seite 60 ist im Gebrauch des Sprechventils 10 distal anordenbar. Die zweite Seite 62 ist im Gebrauch des Sprechventils 10 proximal anordenbar. Das Deckelteil 50 umfasst einen Mantel 52, der auf seiner Innenseite ein Deckelgewinde 58 umfasst. Das Deckelgewinde 58 ist ein eingängiges Innengewinde mit einer Steigung von etwa 0,75 der Höhe 59 des Deckelgewindes 58. Weiterhin umfasst das Deckelteil 50 eine Ringwandung 54, die über einen als Fase ausgestalteten Übergang 53 mit dem Mantel verbunden ist. Die Ringwandung 54 umfasst zentral eine Atemöffnung 56. Weiterhin umfasst die Ringwandung 54 einen Ventilsitz 57, der auf der zweiten Seite 62 der Ringwandung 54 angeordnet ist. Der Ventilsitz umgibt die Atemöffnung 56.

Das Gehäuse 20 umfasst des Weiteren eine das Einsteckteil 30. Diese umfasst eine erste Seite 22 und eine dieser gegenüber liegende zweite Seite 24. Die erste Seite 22 ist im Gebrauch des Sprechventils 10 distal anordenbar. Die zweite Seite 24 ist im Gebrauch des Sprechventils 10 proximal anordenbar. Das Einsteckteil 30 umfasst einen Steckabschnitt 32 und einen Gewindeabschnitt 36. Der Gewindeabschnitt 36 umfasst einen Gewindegang 38 mit einer Höhe 37 und einer metrischen Gewindesteigung 39 von etwa 0,75-mal der Höhe 37. Der zweiten Seite 24 zugeordnet umfasst das Einsteckteil 30 einen Steckabschnitt 32, der die Funktion eines 15 mm oder 22 mm Konnektors übernimmt. Mit dem Steckabschnitt 32 ist das Sprechventil 10 mit einem nicht dargestellten Tracheostomahilfsmittel verbindbar. Zwischen dem Steckabschnitt 32 und dem Gewindeabschnitt 36 ist ein Anschlag 34 angeordnet, der sowohl ein Aufschrauben des Deckelteils 50 auf den Gewindeabschnitt 36 als auch ein Einstecken des Steckabschnittes 32 in ein Tracheostomahilfsmittel begrenzt.

Das Einsteckteil 30 umfasst eine Filterausnehmung 40, in die ein Filter 70 aufgenommen werden kann. Die Filterausnehmung 40 ist zur zweiten Seite 24 hin durch einen Respirationsschutz 42 begrenzt. Der Respirationsschutz 42 umfasst eine Anzahl von Stegen, die ein Einatmen des Filters 70 verhindern. Der ersten Seite 22 zugeordnet ist eine Verschlussmittelaufnahme 44 in einer die Filterausnehmung 40 begrenzenden Innenwandung 43 angeordnet, die als umlaufende Kerbe ausgestaltet ist. Ein Verschlussmittel 80 kann in die Verschlussmittelaufnahme 44 einrasten. Hierzu umfasst das Verschlussmittel an einem Außenring 80 einen Wulst 88.

Wird das Deckelteil 50 auf das Einsteckteil 30 geschraubt, so greift der Gewindeabschnitt 36 in das Deckelgewinde 58 ein. Mittels Drehen des Deckelteils 50 auf dem Einsteckteil 30 kann folglich ein Abstand des Ventilsitzes 57 zum Verschlussmittel 80, das in dem Einsteckteil 30 arretiert ist, verändert werden. Wie in der allgemeinen Beschreibung erwähnt, kann der Gewindeabschnitt 36 und/oder das Deckelgewinde 58 einen Anschlag aufweisen, der ein Drehen des Deckelteils 50 auf dem Einsteckteil 30 begrenzt und insbesondere ein versehentliches Trennen des Deckelteils 50 von dem Einsteckteil 30 verhindert. Ausgestaltungen des Anschlages sind beispielhaft oben beschrieben.

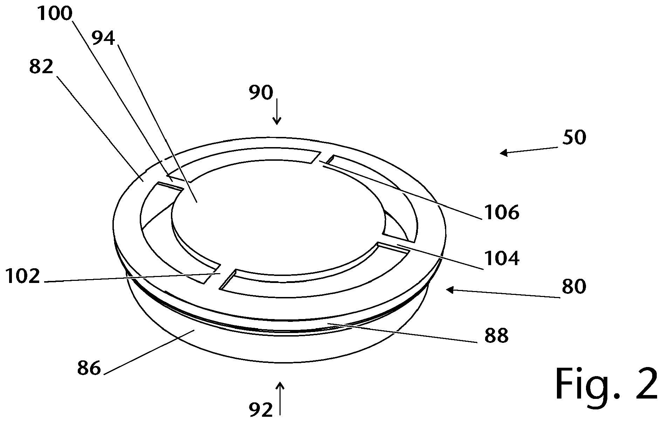

Fig. 2 zeigt eine Detailaufnahme des Verschlussmittels 80 aus Fig. 1. Das Verschlussmittel 80 umfasst eine erste Seite 90 und eine zweite Seite 92. Die erste Seite 90 ist im Gebrauch des Sprechventils 10 distal anordenbar. Die zweite Seite 92 ist im Gebrauch des Sprechventils 10 proximal anordenbar. Das Verschlussmittel 80 umfasst einen Außenring 82, der auf seinem Ringmantel 86 einen umlaufenden Wulst 88 umfasst. Ein Dichtmittel 94 ist in einer neutralen Position konzentrisch im Außenring 82 angeordnet. Gehalten wird das Dichtmittel 94 von hier beispielhaft vier Aufhängungen 100, 102, 104, 106. In weiteren beispielhaften Ausgestaltungen, die in der allgemeinen Beschreibung erwähnt sind, können auch mehr oder weniger Aufhängungen vorgesehen sein beziehungsweise die Ausgestaltung der Aufhängungen von der in der Fig. 2 gezeigten Ausgestaltung abweichen.

Im Gebrauch des Sprechventils 10 wird beim Ausatmen ein Druck von der zweiten Seite 92 aufgebaut, der das Dichtmittel 94 zur ersten Seite 90 hin deplatziert. Diese Deplatzierung ist größer, wenn der Benutzer versucht zu sprechen, da natürlicherweise der Ausatemdruck beim Sprechen größer ist. Ist das Deckelteil 50 so weit auf das Einsteckteil 30 aufgeschraubt, dass das Dichtmittel 94 dabei zur Anlage an den Ventilsitz 57 kommt, wird das Sprechventil 10 verschlossen und die ausgeatmete Luft kann durch die Larynx des Benutzers oder ein Shunt-Ventil strömen, damit der Benutzer sprechen kann.

Durch das vorgeschlagene Sprechventil 10 wird dem Benutzer vorteilhafterweise ermöglicht freihändig zu sprechen. Weiterhin wird dem Benutzer komfortabel die Möglichkeit gegeben, das Sprechventil 10 auf seine Bedürfnisse beziehungsweise derzeitige Situation anzupassen und den notwendigen Ausatemdruck zur Einleitung des Sprechens zu verstellen.

## Patentansprüche

1. Sprechventil (10) zumindest umfassend ein Gehäuse (20) und ein Verschlussmittel (80), wobei das Gehäuse (20) ein Einsteckteil (30) und ein haubenförmiges oder domförmiges Deckelteil (50) mit einer Atemöffnung (56) umfasst, wobei das Deckelteil (50) auf dem Einsteckteil (30) anordenbar ist, wobei das Verschlussmittel (80) einen Außenring (82), ein Dichtmittel (94) und zumindest eine Aufhängung (100, 102, 104, 106) umfasst, wobei das Dichtmittel (94) mittels der Aufhängung (100, 102, 104, 106) am Außenring (82) angeordnet ist, wobei eine Innenwandung des Deckelteils (50) ein Deckelgewinde (58) umfasst und das Deckelteil (50) einen Ventilsitz (57) zur Anlage des Dichtmittels (94) ausbildet und mittels des Dichtmittels (94) die Atemöffnung (56) verschließbar ist.

2. Sprechventil (10) gemäß Anspruch 1, wobei die zumindest eine Aufhängung (100, 102, 104, 106) stegförmig ausgestaltet ist.

3. Sprechventil (10) nach einem oder mehreren der vorhergehenden Ansprüche, wobei mittels der Aufhängung (100, 102, 104, 106) das Dichtmittel (94) gummielastisch am Außenring (82) aufgehängt ist.

4. Sprechventil (10) gemäß einem oder mehreren der vorhergehenden Ansprüche, wobei das Verschlussmittel (80) zumindest teilweise in einer zumindest teilweise umlaufenden Verschlussmittelaufnahme (44) des Gehäuses (20) anordenbar ist.

5. Sprechventil (10) gemäß einem oder mehreren der vorhergehenden Ansprüche, wobei das Einsteckteil (30) einen Gewindeabschnitt (36) mit einem Gewindegang (38) umfasst und das Deckelteil (50) ein Deckelgewinde (58) umfasst, wobei der Gewindegang (38) des Einsteckteils (30) und das Deckelgewinde (58) miteinander korrespondierend ausgestaltet sind.

6. Sprechventil (10) gemäß Anspruch 5, wobei der Gewindegang (38) und das Deckelgewinde (58) etwa 0,5 bis etwa 2 Umdrehungen aufweisen.

7. Sprechventil (10) gemäß einem oder mehreren der Ansprüche 5 bis 6, wobei der Gewindegang (38) und das Deckelgewinde (58) eine metrische Gewindesteigung (39) von etwa 0,5 bis etwa 2 Mal einer Höhe (37) des Gewindeabschnittes (36) ist.

8. Sprechventil (10) gemäß einem oder mehreren der vorhergehenden Ansprüche, wobei mittels Drehen des Deckelteils (50) auf dem Einsteckteil (30) ein Abstand des Verschlussmittels (80) von dem Ventilsitz (57) veränderbar ist.

9. Sprechventil (10) gemäß einem oder mehreren der vorhergehenden Ansprüche, wobei dieses einen Filter (70) umfasst, der in dem Einsteckteil (30) angeordnet ist, wobei das Einsteckteil (30) eine Filterarretierung umfasst, mit der der Filter (70) in dem Einsteckteil (30) gehalten ist.

10. Verfahren zur Einstellung eines Verschlussdruckes eines Sprechventils (10) nach einem oder mehreren der Ansprüche 1 bis 9, wobei mittels Drehen des Deckelteils (50) auf dem Einsteckteil (30) ein Abstand zwischen Dichtmittel (94) und dem Ventilsitz (57) verändert wird.

## Claims

1. Speaking valve (10) at least comprising a housing (20) and a closing means (80), wherein the housing (20) comprises an insertion part (30) and a hood-shaped or dome-shaped cover part (50) with a breathing opening (56), wherein the cover part (50) can be arranged on the insertion part (30), wherein the closing means (80) comprises an outer ring (82), a sealing means (94) and at least one mounting (100, 102, 104, 106), wherein the sealing means (94) is attached to the outer ring (82) by means of the mounting (100, 102, 104, 106), wherein an inner wall of the cover part (50) comprises a cover thread (58) and the cover part (50) forms a valve seat (57) for the abutment of the sealing means (94) and the breathing opening (56) can be closed by means of the sealing means (94).

2. Speaking valve (10) according to claim 1, wherein the at least one mounting (100, 102, 104, 106) is web-shaped.

3. Speaking valve (10) according to one or more of the preceding claims, wherein by means of the mounting (100, 102, 104, 106) the sealing means (94) is rubber-elastically mounted on the outer ring (82).

4. Speaking valve (10) according to one or more of the preceding claims, wherein the closing means (80) is at least partially disposable in an at least partially circumferential closing means receptacle (44) of the housing (20).

5. Speaking valve (10) according to one or more of the preceding claims, wherein the insertion part (30) comprises a threaded portion (36) having a thread (38) and the cover part (50) comprises a cover thread (58), wherein the thread (38) of the insertion part (30) and the cover thread (58) are configured to correspond with each other.

6. Speaking valve (10) according to claim 5, wherein the thread (38) and the cover thread (58) have about 0.5 to about 2 turns.

7. Speaking valve (10) according to one or more of claims 5 to 6, wherein the thread (38) and the cover thread (58) is a metric thread pitch (39) of about 0.5 to about 2 times a height (37) of the threaded portion (36).

8. Speaking valve (10) according to one or more of the preceding claims, wherein a distance of the closing means (80) from the valve seat (57) can be varied by means of turning the cover part (50) on the insertion part (30).

9. Speaking valve (10) according to one or more of the preceding claims, wherein it comprises a filter (70) disposed in the insertion part (30), wherein the insertion part (30) comprises a filter lock for retaining the filter (70) in the insertion part (30).

10. Method for adjusting a closing pressure of a speaking valve (10) according to one or more of claims 1 to 9, wherein a distance between the sealing means (94) and the valve seat (57) is varied by means of turning the cover part (50) on the insertion part (30).

## Revendications

1. Valve de phonation (10) comprenant au moins un boîtier (20) et un moyen de fermeture (80), ledit boîtier (20) comprenant une partie d'insertion (30) et une partie de couvercle (50) en forme de capot ou de dôme avec une ouverture de respiration (56), ladite partie de couvercle (50) étant disposable sur ladite partie d'insertion (30), ledit moyen de fermeture (80) comprenant une bague extérieure (82), un moyen d'étanchéité (94) et au moins une suspension (100, 102, 104, 106), ledit moyen d'étanchéité (94) étant disposé sur ladite bague extérieure (82) au moyen de ladite suspension (100, 102, 104, 106), une paroi intérieure de ladite partie couvercle (50) comprenant un filetage (58) de couvercle et ladite partie couvercle (50) formant un siège de vanne (57) pour l'application dudit moyen d'étanchéité (94) et ladite ouverture de respiration (56) étant fermable au moyen dudit moyen d'étanchéité (94).

2. Valve de phonation (10) selon la revendication 1, dans laquelle ladite au moins une suspension (100, 102, 104, 106) est configurée en forme de barrette.

3. Valve de phonation (10) selon une ou plusieurs des revendications précédentes, dans laquelle, au moyen de ladite suspension (100, 102, 104, 106), ledit moyen d'étanchéité (94) est suspendu à ladite bague extérieure (82) avec une élasticité de type caoutchouc.

4. Valve de phonation (10) selon une ou plusieurs des revendications précédentes, dans laquelle ledit moyen de fermeture (80) est disposable au moins partiellement dans un logement (44) du moyen de fermeture au moins partiellement périphérique dudit boîtier (20).

5. Valve de phonation (10) selon une ou plusieurs des revendications précédentes, dans laquelle ladite partie d'insertion (30) comprend une section filetée (36) avec un filet (38) et ladite partie de couvercle (50) comprend un filetage (58) de couvercle, ledit filet (38) de ladite partie d'insertion (30) et ledit filetage (58) de couvercle étant configurés de manière à se correspondre.

6. Valve de phonation (10) selon la revendication 5, dans laquelle ledit filet (38) et ledit filetage (58) de couvercle ont environ 0,5 à environ 2 tours.

7. Valve de phonation (10) selon une ou plusieurs des revendications 5 à 6, dans laquelle ledit filet (38) et ledit filetage (58) de couvercle sont un pas de filet (39) métrique d'environ 0,5 à environ 2 fois une hauteur (37) de ladite section filetée (36).

8. Valve de phonation (10) selon une ou plusieurs des revendications précédentes, dans laquelle une distance entre ledit moyen de fermeture (80) et ledit siège de vanne (57) est modifiable en faisant tourner ladite partie de couvercle (50) sur ladite partie d'insertion (30).

9. Valve de phonation (10) selon une ou plusieurs des revendications précédentes, comprenant un filtre (70) disposé dans ladite pièce d'insertion (30), ladite pièce d'insertion (30) comprenant un dispositif de blocage de filtre avec lequel ledit filtre (70) est maintenu dans ladite pièce d'insertion (30).

10. Procédé de réglage d'une pression de fermeture d'une vanne de phonation (10) selon une ou plusieurs des revendications 1 à 9, dans lequel une distance entre ledit moyen d'étanchéité (94) et ledit siège de vanne (57) est modifié en faisant tourner ladite partie de couvercle (50) sur ladite partie d'insertion (30).
